Europäisches Patentamt

European Patent Office

Office européen des brevets

(11) Veröffentlichungsnummer: **0 245 575**
**A1**

## (12) EUROPÄISCHE PATENTANMELDUNG

(21) Anmeldenummer: 86810526.3

(22) Anmeldetag: 17.11.86

(51) Int. Cl.⁴: **A47G 19/16** , B65D 81/34 , B65D 81/32 , A47J 31/08

(30) Priorität: 12.05.86 CH 1919/86

(43) Veröffentlichungstag der Anmeldung: 19.11.87 Patentblatt 87/47

(84) Benannte Vertragsstaaten: AT BE CH DE ES FR GB GR IT LI LU NL SE

(71) Anmelder: Greither, Peter Hausen CH-9533 Kirchberg(CH)

(72) Erfinder: Greither, Peter Hausen CH-9533 Kirchberg(CH)

(74) Vertreter: Wenger, René et al Hepp & Partner AG Marktgasse 18 CH-9500 Wil(CH)

(54) Filterkörper, insbesondere mit Tee oder dergleichen Stoffen gefüllter Filterbeutel.

(57) Der Filterkörper (I) ist neben dem Extraktionsgut (2), das beispielsweise aus Tee oder dergleichen besteht, auch noch mit einem Körper (3) gefüllt, der bestimmte Wirkstoffe enthält. Diese Wirkstoffe werden erst unter der Einwirkung des Extraktionsmittels, also z.B. beim Uebergiessen mit heissem Wasser freigesetzt. Der Körper (3) ist vorzugsweise eine Gelatinekapsel.

Fig. 1

EP 0 245 575 A1

## Filterkörper, insbesondere mit Tee oder dergleichen Stoffen gefüllter Filterbeutel

Die Erfindung betrifft einen Filterkörper zur Aufnahme eines festen Extraktionsgutes, insbesondere einen mit Tee und dergleichen Stoffen gefüllten Filterbeutel. Derartige Filterkörper haben sich für die schnelle Zubereitung von Tee seit vielen Jahren bewährt. Ein Nachteil bisher bekannter Teebeutel besteht insbesondere darin, dass bestimmte Aromastoffe sich verhältnismässig rasch verflüchtigen. Gerade diese Stoffe wie z.B. ätherische Oele usw. sind es jedoch, welche dem Getränk einen charakteristischen Geschmack geben.

Um eine zu rasche Verdunstung bestimmter Aromastoffe zu vermeiden, ist es in bestimmten Fällen üblich, die Filterbeutel einzeln in einer aromadichten Aussenhüele zu verschweissen. Diese Massnahme ist jedoch relativ aufwendig, da die Verpackungskosten insgesamt in keinem Verhältnis mehr stehen zum Wert des verpackten Tees. Ausserdem ist die Aussenverpackung nur beschränkt wirksam, da eine Verdunstung in einem gewissen Umfang auch innerhalb des hermetisch verschweissten Beutels stattfindet.

Es ist daher einerseits eine Aufgabe der Erfindung, einen Filterbeutel für Tee und dergleichen zu schaffen, welcher Aromastoffe enthält, die erst unmittelbar bei der Extraktion bzw. bei der Zubereitung des Getränks durch Uebergiessen mit heissem Wasser freigesetzt werden. Andererseits ist es aber auch eine Aufgabe der Erfindung, einen Filterkörper zu schaffen, der es erlaubt, die Zubereitung eines Getränks mit der Applikation eines bestimmten Wirkstoffes zu verbinden. Diese Aufgabe wird erfindungsgemäss durch einen Filterkörper gelöst, der die Merkmale von Patentanspruch I aufweist.

Da der Wirkstoff erst unter Einwirkung des Extraktionsmittels freigesetzt wird, ist es möglich, dem Extraktionsgut wie z.B. Tee oder Kaffee einen Aromastoff beizugeben, so dass auch nach längerer Lagerung des Filterkörpers mit dem Extraktionsgut unmittelbar bei der Zubereitung ein Getränk gewonnen wird, bei dem auch die normalerweise nur bei Frischprodukten vorhandenen Aromastoffe anzutreffen sind. Alternativ oder in Kombination mit bestimmten Aromastoffen kann der Körper aber auch pharmazeutische Wirkstoffe enthalten. Viele Medikamente mussen ohnehin zusammen mit einer Flüssigkeit eingenommen werden. Der erfindungsgemässe Filterkörper vereinfacht und erleichtert daher die Einnahme eines Medikaments auf überraschend einfache Weise.

Der Körper ist vorzugsweise eine mit dem Wirkstoff und/oder dem Zusatzstoff gefüllte Gelatinekapsel, wobei es sich um eine Hartgelatine-Kapsel oder um eine Weichgelatine-Kapsel handeln kann. Die Gelatinekapsel erlaubt nicht nur die Aufnahme fester, sondern auch pastenförmiger oder flüssiger Wirkstoffe. Dadurch können beispielsweise ätherische Oele dem Extraktionsgut beigemengt werden, ohne dass auch bei längerer Lagerung eine Verdunstung befürchtet werden muss. Die Gelatinekapsel löst sich unter der Einwirkung von heissem Wasser auf und ist absolut geschmacksneutral. Alternativ zur Unterbringung in einer Gelatinekapsel könnten die Wirkstoffe selbstverständlich auch in der Form von Tabletten, Pellets, Dragées oder dergleichen dem Extraktionsgut beigemengt werden.

Als Filterkörper kann beispielsweise ein an sich bekannter Doppelkammerbeutel verwendet werden. Anlagen für die Herstellung und Füllung derartiger Doppelkammerbeutel sind bei den Abfüllern bereits vorhanden und müssen lediglich noch modifiziert werden. Selbstverständlich ist auch jede andere Form von Filterkörper denkbar, wie z.B. geschweisste rechteckige Papierfilterbeutel, genähte Stoffbeutel usw. Es wäre sogar denkbar, dass der Filterkörper ein einseitig geöffneter Filterkörper ist, wie er beispielsweise durch die Filtertüten für die Zubereitung von Kaffee bekannt geworden ist. Eine derartige Filtertüte könnte eine separate Kammer fur die Aufnahme des Körpers mit den Wirkstoffen enthalten.

Ausführungsbeispiele der Erfindung sind in den Zeichnungen dargestellt und werden nachstehend genauer beschrieben. Es zeigen:

Figur I einen geschlossenen Filterbeutel mit einem dem Extraktionsgut beigemengten Körper,

Figur 2 einen Querschnitt durch einen Hartgelatine-Kapsel, und

Figur 3 eine Filtertüte mit einem in einer separaten Kammer angeordneten Körper.

Figur I zeigt einen Filterkörper I in der Form eines an sich bekannten Doppelkammerbeutels mit den beiden Kammern 4 und 4'. Diese beiden Kammern sind mit einer Heftklammer 5 miteinander verbunden, welche auch die Schnur 6 festhält, an der eine Etikette 7 mit einer beliebigen Beschriftung befestigt ist. In der teilweise aufgeschnitten dargestellten Kammer 4 ist das Extraktionsgut 2 sichtbar, das beispielsweise aus Teeblättern bestehen kann. Das Extraktionsgut wird normalerweise ein loses Schüttgut sein, dem wie dargestellt ein Körper 3 beigemengt ist, der bestimmte Wirkstoffe enthält, die erst bei der Extraktion freigesetzt werden. In bestimmten Fällen kann der Körper auch Zusatzstoffe enthalten, welche beispielsweise als Trägerstoff oder Lösungsmittel für den Wirkstoff dienen können. Auch Mischungen mehrerer Wirkstoffe und/oder Zusatzstoffe sind denkbar. Der Körper 3 kann eine Gelatinekapsel oder eine gepresste Tablette oder dergleichen sein.

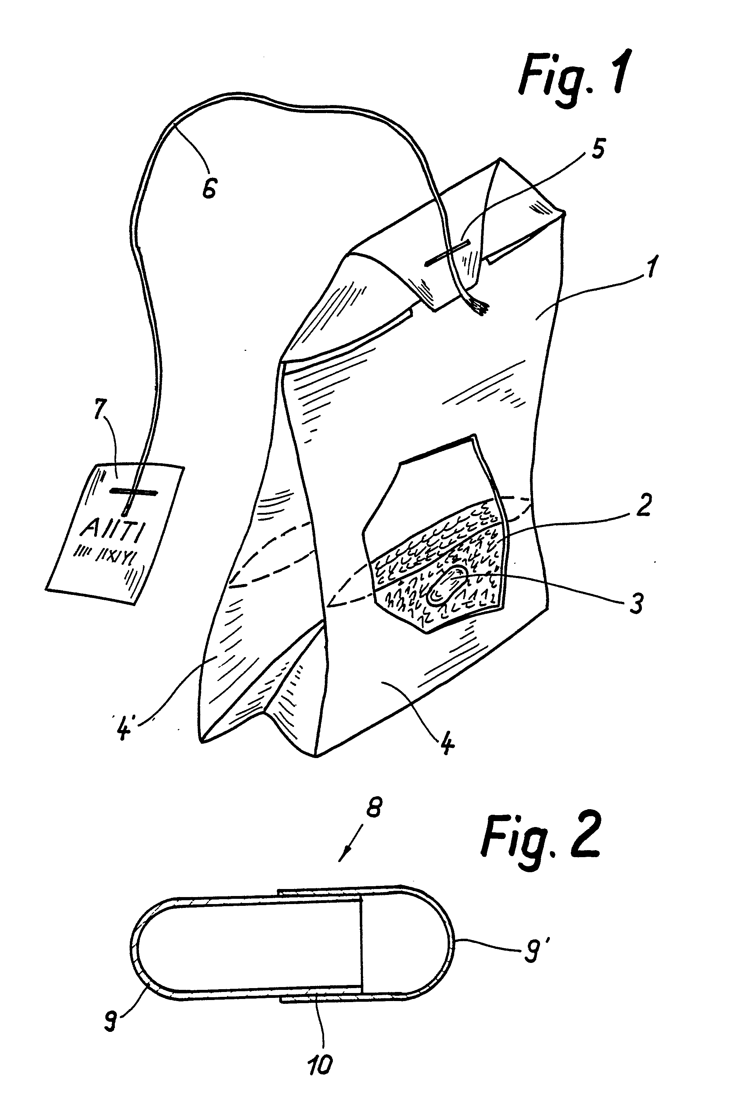

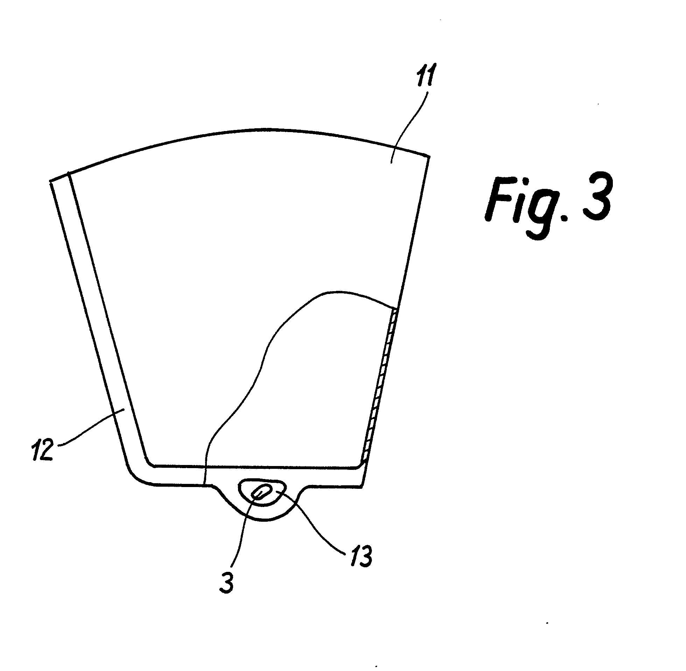

Bei der Verwendung einer Gelatinekapsel, wie sie beispielsweise in Figur 2 dargestellt ist, besteht die Möglichkeit, auch flüssige oder pastenförmige Wirkstoffe beizugeben. Figur 2 zeigt beispielsweise eine Hartgelatinekapsel 8 bestehend aus den beiden Teilschalen 9 und 9'. Diese Teilschalen sind an der Versiegelungsstelle l0 miteinander verbunden und bilden somit eine hermetisch dichte Kapsel. Neuerdings können Hartgelatinekapseln auch mit flüssigen oder pastenförmigen Substanzen gefüllt werden. Das Herstellen und Füllen von Weich-oder Hartgelatine-Kapseln ist dem Fachmann bekannt und wird daher hier nicht näher erläutert.

Wie in Figur 3 dargestellt, muss es sich beim Filterkörper nicht zwangsläufig um einen geschlossenen Beutel handeln. Die Figur zeigt eine Filtertüte ll, wie sie z.B. für die Zubereitung von Kaffee verwendet werden kann. Das Extraktionsgut wird somit erst bei der Zubereitung in den Filterkörper eingegeben. Die Filtertüte ll weist eine Schweissnaht l2 auf, die am unteren Bereich erweitert ist und eine Kammer l3 zur Aufnahme des Körpers 3 bildet. Derart könnten auch bei der traditionellen Kaffeezubereitung mittels Papierfilter bestimmte Aromastoffe oder andere Wirkstoffe in den Extrakt freigesetzt werden.

## Ansprüche

l. Filterkörper (l) zur Aufnahme eines festen Extraktionsgutes (2), insbesondere mit Tee und dergleichen Stoffen gefüllter Filterbeutel, dadurch gekennzeichnet, dass er wenigstens einen Körper (3) enthält, der unter Einwirkung des Extraktions-Mittels wenigstens einen Wirkstoff und/oder einen Zusatzstoff freisetzt.

2. Filterkörper nach Anspruch l, dadurch gekennzeichnet, dass der Körper eine mit dem Wirkstoff und/oder Zusatzstoff gefüllte Gelatine-Kapsel ist.

3. Filterkörper nach Anspruch 2, dadurch gekennzeichnet, dass die Gelatinekapsel eine Hartgelatine-Kapsel ist.

4. Filterkörper nach Anspruch 2, dadurch gekennzeichnet, dass die Gelatinekapsel eine Weichgelatine-Kapsel ist.

5. Filterkörper nach Anspruch l, dadurch gekennzeichnet, dass der Wirkstoff und/oder der Zusatzstoff in Tablettenform, zu Pellets, zu Granulat oder dergleichen gepresst ist.

6. Filterkörper nach einem der Ansprüche l bis 4, dadurch gekennzeichnet, dass der Wirkstoff und/oder der Zusatzstoff eine Flussigkeit ist.

7. Filterkörper nach einem der Ansprüche l bis 6, dadurch gekennzeichnet, dass der Wirkstoff ein Aromastoff oder ein Farbstoff ist.

8. Filterkörper nach einem der Ansprüche l bis 6, dadurch gekennzeichnet, dass der Wirkstoff ein Pharmazeutika ist.

9. Filterkörper nach einem der Ansprüche l bis 8, dadurch gekennzeichnet, dass er ein Doppelkammer-Beutel ist.

*Fig. 1*

*Fig. 2*

Fig. 3

## DOCUMENTS CONSIDERES COMME PERTINENTS

| Catégorie | Citation du document avec indication, en cas de besoin. des parties pertinentes | Revendication concernée | CLASSEMENT DE LA DEMANDE (Int. Cl.4) |
|---|---|---|---|
| X | FR-A-1 503 185  (N.V. UNILEVER)<br><br>* Page 1, lignes 25-31; Figures *<br><br>--- | 1,2,6, 7 | A 47 G   19/16<br>B 65 D   81/34<br>B 65 D   81/32<br>A 47 J   31/08 |
| X | CH-A-  237 897  (RIKLI-EGGER AG)<br><br>* En entier *<br><br>--- | 1,5,7, 9 | |
| X | DE-A-2 500 100  (KOCAMAZ)<br><br>*  Page  1,  ligne  17  - page 2, ligne 21 *<br><br>--- | 1,5,7, 9 | |
| X | CH-A-  490 024  (GENERAL FOODS CORP.)<br>* Revendication 1 *<br><br>--- | 1,5 | |
| A | DE-C-  239 057  (KACHELLEK)<br><br>* En entier *<br><br>--- | 1,2,6, 7 | |
| A | US-A-3 287 806  (KELLERMAN)<br>* Colonne 2, lignes 11-16 *<br><br>----- | 6-8 | |

DOMAINES TECHNIQUES RECHERCHES (Int. Cl.4)

A 47 G
B 65 D

Le présent rapport de recherche a été établi pour toutes les revendications

| Lieu de la recherche | Date d'achèvement de la recherche | Examinateur |
|---|---|---|
| LA HAYE | 30-01-1987 | BEUGELING G.L.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82